(19) Europäisches Patentamt / European Patent Office / Office européen des brevets

(11) **EP 2 983 691 B1**

(12) **FASCICULE DE BREVET EUROPEEN**

(45) Date de publication et mention
de la délivrance du brevet:
**13.12.2017 Bulletin 2017/50**

(21) Numéro de dépôt: **14716809.0**

(22) Date de dépôt: **10.04.2014**

(51) Int Cl.:
*A61K 38/05* (2006.01)     *A61K 36/899* (2006.01)
*A61K 31/728* (2006.01)     *A61P 17/02* (2006.01)
*A61K 9/00* (2006.01)     *A61K 9/107* (2006.01)

(86) Numéro de dépôt international:
**PCT/EP2014/057224**

(87) Numéro de publication internationale:
**WO 2014/167039 (16.10.2014 Gazette 2014/42)**

(54) **ASSOCIATION SYNERGIQUE DE L'ALANINE-GLUTAMINE, L'ACIDE HYALURONIQUE ET UN EXTRAIT D'AVOINE ET SON UTILISATION DANS UNE COMPOSITION DESTINEE A LA CICATRISATION ET LA REPARATION DES LESIONS CUTANEES**

SYNERGISTISCHE KOMBINATION VON ALANIN-GLUTAMIN, HYALURONSÄURE UND EINEM HAFEREXTRAKT UND VERWENDUNG DAVON IN EINER ZUSAMMENSETZUNG ZUR WUNDHEILUNG UND ZUR REPARATUR VON HAUTVERLETZUNGEN

SYNERGISTIC COMBINATION OF ALANINE-GLUTAMINE, HYALURONIC ACID AND AN OAT EXTRACT AND THE USE THEREOF IN A COMPOSITION INTENDED FOR HEALING WOUNDS AND REPAIRING SKIN LESIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.04.2013 FR 1353231**

(43) Date de publication de la demande:
**17.02.2016 Bulletin 2016/07**

(73) Titulaire: **Pierre Fabre Dermo-Cosmétique
92100 Boulogne-Billancourt (FR)**

(72) Inventeurs:
• **CASTEX-RIZZI, Nathalie
F-31770 Colomiers (FR)**

• **DUPLAN, Hélène
F-31320 Auzeville Tolosan (FR)**
• **DECHELETTE, Corinne
F-81800 Rabastens (FR)**
• **BONZOM, Laetitia
F-31500 Toulouse (FR)**

(74) Mandataire: **Regimbeau
20, rue de Chazelles
75847 Paris Cedex 17 (FR)**

(56) Documents cités:
**FR-A1- 2 926 461     FR-A1- 2 977 494
US-B1- 6 368 579**

EP 2 983 691 B1

**Description**

La présente invention concerne une association synergique comprenant le dipeptide L-alanyl-L-glutamine, l'acide hyaluronique ou un de ses sels et un extrait d'avoine, avantageusement destinée à la cicatrisation et la réparation des lésions cutanées.

La cicatrisation est un ensemble de phénomènes locaux de défense, survenant après une agression : blessure, brûlure, acte dermatologique, intervention chirurgicale. De nombreux produits actifs en provenance du sang et du tissu sont libérés au cours de ces phénomènes : enzymes, protéines diverses, histamine, etc. La cicatrisation comprend plusieurs étapes, dont la première est la coagulation du sang, arrêtant le saignement. Les globules blancs venant du sang éliminent les cellules mortes. Puis des cellules survivantes prolifèrent et donnent naissance à un nouveau tissu, dont l'aspect dépend de la localisation de la lésion.

La cicatrisation dépend de plusieurs facteurs, notamment nutritionnels, métaboliques, endocriniens ou médicamenteux ; on observe des retards de cicatrisation chez les sujets dénutris ou âgés, ou en cas de prise prolongée de corticoïdes. Avec l'âge, le processus d'amélioration de la cicatrisation et de récupération des lésions cutanées devient en effet moins performant, le processus est plus lent.

Il existe toujours un besoin de proposer de nouvelles compositions pour une réparation rapide et esthétique des lésions cutanées.

Les propriétés pharmacologiques du dipeptide L-alanyl-L-glutamine administré par voie orale décrites dans la littérature sont nombreuses.

Entre autres, le dipeptide L-alanyl-L-glutamine est utilisé *per os* comme supplément chez les sportifs pour faciliter la récupération après l'effort et après une intervention chirurgicale pour diminuer la durée de l'hospitalisation.

Ce dipeptide est également indiqué en nutrition parentérale en complément à une solution standard d'acides aminés ou à d'autres mélanges utilisés en nutrition parentérale chez des patients de réanimation nécessitant un apport en glutamine.

Dans la présente invention, les inventeurs ont montré de manière surprenante que le dipeptide L-alanyl-L-glutamine utilisé par voie topique possède une activité biologique sur la peau, basée sur ses propriétés régénérantes : action sur la migration des kératinocytes.

De plus, les inventeurs ont également montré que cette activité est fortement amplifiée lorsque ce dipeptide est associé à un extrait d'avoine et à de l'acide hyaluronique.

En particulier, les inventeurs ont mis en évidence l'existence d'une synergie entre les trois composants que sont l'extrait d'avoine et plus spécifiquement l'extrait de plantules d'avoine, l'acide hyaluronique ou un des sels et le L-alanyl-L-glutamine sur la migration des kératinocytes. Cette activité est particulièrement intéressante dans la régénération tissulaire et la cicatrisation des lésions cutanées.

En effet, la migration des cellules épithéliales est une étape importante du développement et du processus de réparation tissulaire, tels que l'embryogénèse et la cicatrisation.

Les mécanismes d'initiation, de coordination et d'arrêt des mouvements des cellules, ne sont pas complètement élucidés, cependant, le rôle primordial de la migration cellulaire est bien établi. Plusieurs agents susceptibles de stimuler ce processus cellulaire ont été caractérisés, telles que certaines protéines matricielles ou cytoplasmiques (SANTORO MM., GAUDINO G., Cellular and molecular facets of keratinocyte reepithelization during wound healing. EXP. CELL. RES. 304(1) : 274-286, 2005) (WERNER S., GROSE R. Regulation of wound healing by growth factors and cytokines. PHYSIOL. REV. 83(3) : 835-870, 2003) (STEFFENSEN B., AKKINEN L., LARIAVA H. Proteolytic events of wound healing-coordinated interactions among matrix metallopteinases (MMPs), integrins, and extracellular matrix molecules. CRIT. REV. ORAL BIOL. MED. 12(5) : 373-398, 2001).

Lors de la cicatrisation cutanée et dans les affections inflammatoires chroniques dermatologiques, les kératinocytes sont « activés » pour entreprendre le processus de migration. Les cellules voient alors leur phénotype influencé par les interactions avec la matrice extracellulaire d'une part et par les interactions cellules-cellules, d'autre part (MCMILLAN J.R., AKIYAMA M., SHIMIZU H. Epidermal basement membrane zone component : ultrastructural distribution and molecular interactions. J DERM SC. 31 :169-177, 2003). Les kératinocytes de l'assise basale des berges d'une plaie, migrent sur la plaie et la recouvrent. En effet, les kératinocytes sont activés au contact de la fibronectine, du collagène dermique interstitiel (type 1), du collagène IV et de la laminine 5 de la lame basale. Ils sont aussi régulés par certains facteurs de croissance polypeptidiques comme le TGF$\beta$, TGF$\alpha$ et l'EGF. De plus des cytokines (IL1, TNF$\alpha$) et chémokines (RANTES et IL8) contribuent aussi à augmenter la vitesse de ré-épithélisation d'une plaie, suite à l'activation kératinocytaire (SZABO I., WETZEL M.A., ROGERS TJ. Cell-Density-Regulated Chemotactic Responsiveness of Keratinocytes In Vitro. J INVEST DERMATOL 117 :1083-1090, 2001.

LEGENDE DE LA FIGURE EN ANNEXE

La Figure unique annexée illustre l'effet de l'extrait de plantules d'avoine / d'acide hyaluronique et de L-alanyl-L-glutamine et de leurs associations sur la migration des kératinocytes.

La présente invention a pour objet une association comprenant de la L-alanyl-L-glutamine, de l'acide hyaluronique ou un de ses sels et un extrait d'avoine.

Le terme « L-alanine » se réfère à l'acide (S)-2-aminopropanoïque, également appelé acide α-aminopropionique. La L-alanine est un acide aminé neutre aux propriétés légèrement apolaires et hydrophobes.

Le terme « L-glutamine » se réfère à l'acide 2-aminoglutaramique. La L-glutamine est un acide aminé semi-essentiel, polaire non chargé et hydrophile.

Dans un mode de réalisation particulier de l'invention, l'association comprend un acide hyaluronique ou un de ses sels, de haut poids moléculaire. De préférence, le poids moléculaire sera compris entre 50 000 et 750 000 Da, et de préférence entre 250 000 et 450 000 Da.

Le sel de l'acide hyaluronique sera préférentiellement le hyaluronate de sodium.

Selon un mode d'exécution de l'invention, le sel hyaluronate aura un poids moléculaire compris entre 50 000 et 750 000 Da, et de préférence entre 250 000 et 450 000 Da.

Dans un mode de réalisation de l'invention, l'extrait d'avoine est obtenu à partir de plantules d'avoine, et préférentiellement tel que décrit dans WO 2010/054879.

Par « plantules d'avoine », on entend au sens de la présente invention l'avoine avant épiaison, c'est-à-dire au stade après germination (environ 2 semaines à 2 mois après germination) durant le stade de la montaison jusqu'à l'épiaison non comprise. On appelle «montaison» la phase de croissance qui correspond à l'élongation de la tige et à la montée de l'épi en formation, avant floraison. Des métabolites secondaires sont décrits dans la demande WO2010/054879 comme composants d'un extrait de plantule d'avoine : les flavonoïdes et les saponines de type avenacoside. Ledit extrait est caractérisé par la présence de 2 à 15% de flavonoïdes et 0,2 à 2% d'avenacosides A et B.

Le procédé de préparation de l'extrait d'avoine peut être comme suit :

- séchage et broyage des parties d'avoine, de préférence les plantules d'avoine,
- extraction en solvant organique choisi dans le groupe constitué des cétones, des esters, des alcools en C1 à C4 et des mélanges en toute proportion miscibles de ces solvants, et
- centrifugation ou filtration.

Avantageusement, le solvant organique du procédé selon l'invention est choisi dans le groupe constitué de l'acétone, la méthyl éthyl cétone, la méthylisobutyl cétone, l'acétate d'éthyle, un alcool en C1 à C4 et un mélange en toute proportion miscible de ces solvants.

Le marc obtenu par l'étape d'extraction est ensuite séparé de l'extrait par centrifugation ou filtration et la solution peut être plus ou moins concentrée jusqu'à l'obtention d'un extrait sec.

Selon un mode d'exécution de l'invention, un support peut être rajouté lors de l'étape de séchage dans des proportions massiques par rapport à la matière sèche extraite pouvant varier de 1 à 75%. Le support peut être un sucre comme la maltodextrine, le lactose, de la silice ou tout autre support cosmétologiquement acceptable.

Dans un autre mode de réalisation de l'invention, la solution est concentrée de façon à obtenir un mout comprenant de 60 à 80% de matière sèche, et de préférence 70% de matière sèche.

Dans un mode de réalisation particulier, l'association selon l'invention est caractérisée en ce que le ratio massique acide hyaluronique / extrait d'avoine / L-alanyl-L-glutamine est respectivement compris entre 2/1/3 et 2/1/5.

Dans un autre mode d'exécution, le ratio d'acide hyaluronique, de l'extrait de plantules d'avoine et de L-alanyl-L-glutamine est un rapport en poids respectivement de 2/1/4.

Dans un autre mode de réalisation préféré, la composition selon l'invention comprend un ratio acide hyaluronique / extrait de plantules d'avoine / L-alanyl-L-glutamine respectivement de 2/1/4.

Un autre objet de la présente invention concerne une nouvelle composition dermatologique ou cosmétique destinée à accélérer la réparation cutanée afin de rétablir l'intégrité et la qualité de la peau.

La composition selon l'invention comprend en tant que principe actif dermatologique ou cosmétique l'association d'acide hyaluronique, d'extrait d'avoine et de L-alanyl-L-glutamine ci-dessus mentionnée et comprend en outre au moins un excipient dermatologiquement ou cosmétiquement acceptable.

Dans un mode de réalisation préférée de l'invention, la composition est destinée à une application topique.

Les excipients dermatologiquement (pharmaceutiquement) ou cosmétiquement compatibles peuvent être tout excipient parmi ceux connus de l'homme de l'art en vue d'obtenir une composition pour l'application topique sous forme d'un lait, d'une crème, d'un baume, d'une huile, d'une lotion, d'un gel, d'un gel moussant, d'une pommade, d'un spray, etc.

Dans un mode de réalisation préféré, la composition sera sous forme d'une crème, d'une pommade.

Dans un mode de réalisation, la composition selon l'invention comprend au moins un autre principe actif.

Par « principe actif » selon l'invention, on entend toute substance ayant des propriétés dermatologiques ou cosmétiques.

Cet autre principe actif pourra notamment être sélectionné dans le groupe comprenant des agents cicatrisants, apaisants, antiprurit, anti-âge, antirides, anti-radicalaires, anti-UV, des agents stimulant la synthèse de macromolécules dermiques ou le métabolisme énergétique, des agents hydratants, antibactériens, antifongiques, anti-inflammatoires, anesthésiques.

Préférentiellement, on utilisera des agents cicatrisants et/ou apaisants.

Enfin, un autre objet de la présente invention vise l'utilisation des compositions à base de l'association d'acide hyaluronique, d'extrait d'avoine et de L-alanyl-L-glutamine pour le traitement et la cicatrisation des lésions cutanées.

La présente invention porte, en outre, sur une association d'acide hyaluronique, d'extrait de plantules d'avoine et de L-alanyl-L-glutamine selon l'invention pour la préparation d'une composition destinée à favoriser la migration kératinocytaire.

La composition selon l'invention est destinée aux soins des peaux lésées:

- suite à des actes / traitements invasifs : actes chirurgicaux (exérèses, shavings) avec ou sans suture, cryothérapie, laser ablatif, peelings moyens ou forts, mésothérapie, curetage.
- en post traumatique lors de coupures ou brulures superficielles
- suite à des actes superficiels (non invasifs) nécessitant un produit cicatrisant qui accélère la récupération cutanée, utilisable au long cours (jusqu'à réparation complète de la peau).
- suite à une altération extérieure légère: écorchures superficielles, coup de soleil.

Le traitement des lésions de la peau et des muqueuses selon l'invention pourra notamment comprendre le traitement des coupures, des sutures, des écorchures, des éraflures, des égratignures, des cicatrices post-chirurgie ou post-acte de dermatologie esthétique, des brûlures superficielles, des coups de soleil.

La présente invention porte, en outre, sur l'utilisation d'une composition cosmétique selon l'invention destinée à l'amélioration de la cicatrisation et de la réparation cutanée. L'invention sera mieux comprise à la lecture des résultats ci-dessous qui l'illustrent sans en limiter la portée.

EVALUATION PHARMACOLOGIQUE sur la migration des kératinocytes

Ce test avait pour objectif d'évaluer l'effet de l'acide hyaluronique, de l'extrait de plantules d'avoine, de la L-alanyl-L-glutamine et de leurs associations sur la migration des kératinocytes à l'aide du kit de migration cellulaire Oris Cell Migration Assay (Platypus Technologies).

Matériel biologique

La lignée de kératinocytes humains HaCaT, spontanément immortalisés, fréquemment citée comme modèle de référence dans la littérature a été utilisée.

Protocole de migration cellulaire

Le protocole utilisé pour l'étude de la migration cellulaire repose sur l'utilisation d'un kit 96 puits, Oris Cell Migration Assay (Platypus technologies - TEBU), permettant la miniaturisation et la quantification de ce processus cellulaire.

Le principe de ce test consiste à étudier la migration cellulaire vers le centre du puits de la plaque de 96 puits. Il consiste à placer un stoppeur dans des puits, afin de créer une zone de détection de 2 mm de diamètre. Puis de retirer les stoppeurs une fois que les cellules ont bien adhéré à la surface autour de ceux-ci, et ainsi, de permettre aux cellules de migrer vers la zone de détection. Les plaques sans les stoppeurs et avec les actifs sont mises à incuber à 37°C pendant 24 heures dans du DMEM (Dulbecco's Modified Eagle Medium) 0% SVF (sérum de veau foetal). La quantité de cellules situées dans la zone où il y avait le stoppeur est analysée afin d'évaluer la migration des cellules. Un cache permet de visualiser et de comptabiliser uniquement les cellules situées dans cette zone. Pour chaque condition, la moyenne est réalisée sur 6 à 8 puits.

Les cellules ont été incubées dans du milieu sans SVF. Les produits testés

- EGF : 33 ng/ml,
- extrait de plantules d'avoine : 10 ou 30 $\mu$g/ml,

Préparation de l'extrait :

Extraire 10 g de plantules broyées avec 100 ml de solvant d'extraction acétone / eau (80/20) (v/v).
Filtrer et rincer le marc avec le solvant d'extraction.

Evaporer l'acétone et reprendre la phase aqueuse.
Filtrer. Concentration par séchage jusqu'à l'obtention d'un extrait sec.

- hyaluronate de sodium (poids moléculaire 250-450 kDa): 20 ou 60 $\mu$g/ml,
- L alanyl L glutamine : 40 ou 90 $\mu$g/ml.

Analyse des résultats

Les résultats sont exprimés :

- En intensité de fluorescence (IF), proportionnelle à la quantité des cellules ayant migré.
- En pourcentage d'activité par rapport au témoin 0% SVF

$$\frac{\text{IF traité}}{\text{IF témoin 0\% SVF}} \times 100$$

Analyses statistiques

Des analyses statistiques par le test de Dunnett ont été réalisées sur les valeurs brutes de migration. Ce test donne alors des valeurs de « p value » caractérisant la significativité des résultats obtenus pour les différentes conditions. Le degré de significativité a été fixé à

p<0,05 (* significatif)
P<0,01 (** très significatif)
P<0,001 (*** hautement significatif)
P>0,05 (non significatif).

Résultats

Les résultats à différentes concentrations de l'effet de l'extrait de plantule d'avoine, de l'acide hyaluronique et de la L-alanyl-L-glutamine, seuls ou en association sur la migration des kératinocytes sont indexés dans les tableaux 1 et 2 puis représentés sur la figure unique. Les valeurs de l'intensité de fluorescence représentées sur cette courbe correspondent à une moyenne de 6 à 8 mesures effectuées lors d'une expérience représentative de 3 manipulations indépendantes.

En présence de DMEM et 0% SVF, le contrôle positif des expériences EGF induit bien la migration des kératinocytes. Dans ces conditions expérimentales, l'extrait de plantules d'avoine (10 ou 30 $\mu$g/ml) seul et le hyaluronate (20 ou 60 $\mu$g/ml) seul n'ont pas d'effet significatif sur la migration des kératinocytes. Leur association semble en revanche montrer une tendance à l'augmentation de la migration : +45% ou +27% par rapport au témoin en fonction des concentrations testées (tableaux 1 et 2).

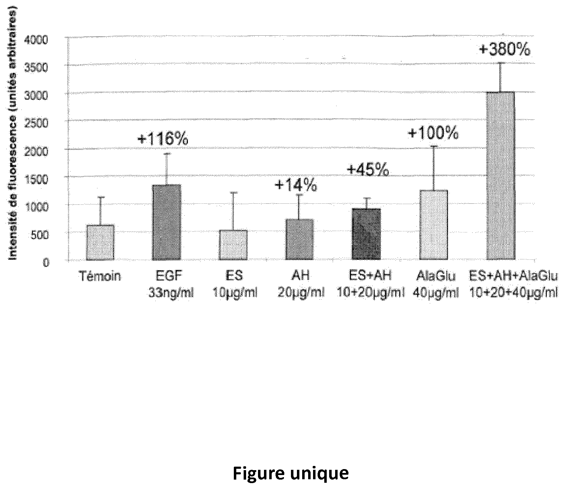

La L-alanyl-L-glutamine seule induit la migration des kératinocytes de façon concentration-dépendante. Ces inductions sont mêmes plus importantes qu'avec le contrôle positif EGF. Les associations des 3 actifs - extrait de plantules d'avoine, acide hyaluronique et L-alanyl-L-glutamine - induisent de façon très importante et statistiquement significative la migration des kératinocytes. Les tableaux 1 et 2 et la figure 1 montrent que ces inductions sont statistiquement significatives par rapport au témoin, mais elles le sont aussi par rapport à l'extrait de plantules d'avoine et à l'acide hyaluronique seuls ou par rapport à leur association.

La Figure 1 annexée illustre l'effet de l'extrait sec de plantules d'avoine (ES), de l'acide hyaluronique (AH) de la L-alanyl L-glutamine (Ala-Glu) et leurs associations sur la migration des kératinocytes. Les pourcentages correspondent aux pourcentages d'activité supplémentaires par rapport au témoin.

Tableau 1 : Effets de l'extrait sec de plantules d'avoine (ES), de l'acide hyaluronique (AH), de la L-alanyl-L-glutamine (A-G) et leurs associations sur la migration des kératinocytes. DS : Déviation standard, **p<0.01 versus témoin.

| Groupes | Concentration | Moyenne $\pm$ DS | % Migration |
|---|---|---|---|
| Témoin | | 1850 $\pm$ 504 | 100 |

(suite)

| Groupes | Concentration | Moyenne ± DS | % Migration |
|---|---|---|---|
| EGF | 33 ng/ml | 2569 ± 554 | 216 |
| Extrait sec de plantule d'avoine (ES) | 10 µg/ml | 1748 ± 681 | 84 |
| Acide hyaluronique (AH) | 20 µg/ml | 1939 ± 438 | 114 |
| ALANYL GLUTAMINE (Ala-Glu) | 40 µg/ml | 2471 ± 788 | 200 |
| ES + AH | 10 + 20 µg/ml | 2129 ± 190 | 145 |
| ES + AH + A-G | 10 + 20 + 40 µg/ml | 4211 ± 541 | 480 ** |

Tableau 2 : Effets de l'extrait sec de plantules d'avoine (ES), de l'acide hyaluronique (AH), de la L-alanyl-L-glutamine (A-G), et leurs associations sur la migration des kératinocytes. DS : Déviation standard, *p<0.05, **p<0.01 versus témoin.

| Groupes | Concentration | Moyenne ± DS | % Migration |
|---|---|---|---|
| Témoin | | 1729 ± 384 | 100 |
| EGF | 33 ng/ml | 3553 ± 1556 | 301 * |
| ES | 30 µg/ml | 1386 ± 226 | 62 |
| AH | 60 µg/ml | 1735 ± 488 | 101 |
| A-G | 90 µg/ml | 2586 ± 1092 | 194 |
| ES + AH | 30 + 60 µg/ml | 1152 ± 541 | 127 |
| ES + AH + A-G | 30 + 60 + 90 µg/ml | 2877 ± 1024 | 317 ** |

Par conséquent une association d'extrait de plantules d'avoine, d'acide hyaluronique ou un de sels et de L-alanyl-L-glutamine augmente de façon synergique la migration des kératinocytes. Ces résultats confirment l'intérêt d'utiliser une telle association dans une composition dermatologique ou cosmétique cicatrisante.

EXEMPLE DE COMPOSITION : émulsion huile dans eau

| | | Pourcentage | Fonction |
|---|---|---|---|
| Phase aqueuse | Eau | QSP100% | |
| | Extrait d'avoine obtenu suite à une extraction acétonique, filtration, et concentration jusqu'à obtention d'un mout à 70% de matière sèche | 0.1 - 3% | Actif |
| | hyaluronate de sodium de poids moléculaire 250-400kDa | 0.05 - 1% | Actif |
| | L-Alanyl-L-glutamine | 0.1 - 1% | Actif |
| | Glycérine | 1 - 30% | Humectant |
| | Hexylène glycol | 0.1 - 7% | Glycol |
| Phase lipophile | Cétéaryl glucoside | 0.1 - 1% | Emulsionnant |
| | Cétéaryl alcool | 0.9 - 4% | Emulsionnant |
| | Stearic acid | 0.5 - 4% | Facteur de consistance |

(suite)

|  |  | Pourcentage | Fonction |
|---|---|---|---|
|  | Glycéryl stéarate | 0.1 - 4% | Facteur de consistance |
|  | Huile végétale | 0.1 - 10% | Emollient |
|  | Butyrospermum ParkII (Shea) Butter | 0.1 - 10% | Emollient |
|  | Silicone | 0.1 - 5% | Emollient |

**Revendications**

1. Association comprenant de la L-alanyl-L-glutamine, de l'acide hyaluronique ou un de ses sels et un extrait d'avoine.

2. Association selon la revendication 1, **caractérisée en ce que** l'acide hyaluronique se présente sous la forme de fragments de hyaluronate de sodium.

3. Association selon la revendication 2, **caractérisée en ce que** le poids moléculaire des fragments de hyaluronate est compris entre 50 000 et 750 000 Da.

4. Association selon l'une des revendications 1 à 3, **caractérisée en ce que** l'extrait d'avoine est obtenu à partir de plantules d'avoine.

5. Association selon la revendication 4, **caractérisée en ce que** le ratio massique acide hyaluronique / extrait de plantules d'avoine / L-alanyl-L-glutamine est respectivement compris entre 2/1/3 et 2/1/5.

6. Association selon la revendication 5, **caractérisée en ce que** le ratio acide hyaluronique /extrait de plantules d'avoine / L-alanyl-L-glutamine est un rapport en poids respectivement de 2/1/4.

7. Association selon l'une des revendications 1 à 6, pour son utilisation topique destinée à traiter les lésions cutanées en favorisant la migration kératinocytaire.

8. Association selon l'une des revendications 1 à 6, pour son utilisation topique destinée à améliorer la cicatrisation.

9. Composition dermatologique ou cosmétique comprenant à titre de principe actif une association selon l'une des revendications 1 à 6, avec au moins un excipient dermatologiquement ou cosmétiquement acceptable.

10. Composition selon la revendication 9, pour son utilisation dans le traitement des coupures, des sutures, des écorchures, des éraflures, des égratignures, des cicatrices post-chirurgie ou post-acte de dermatologie esthétique, des brûlures superficielles, des coups de soleil.

**Patentansprüche**

1. Zusammensetzung, umfassend L-Alanyl-L-Glutamin, Hyaluronsäure oder eines ihrer Salze und einen Haferextrakt.

2. Zusammensetzung gemäß Anspruch 1, **dadurch gekennzeichnet, dass** die Hyaluronsäure die Form von Natriumhyaluronfragmenten aufweist.

3. Zusammensetzung gemäß Anspruch 2, **dadurch gekennzeichnet, dass** das Molekulargewicht der Hyaluronfragmente zwischen 50.000 und 750.000 Da inbegriffen ist.

4. Zusammensetzung gemäß einem der Ansprüche 1 bis 3, **dadurch gekennzeichnet, dass** der Haferextrakt aus Haferkeimlingen gewonnen wird.

5. Zusammensetzung gemäß Anspruch 4, **dadurch gekennzeichnet, dass** das Masseverhältnis Hyaluronsäure / Haferkeimlingsextrakt / L-Alanyl-L-Glutamin jeweils zwischen 2/1/3 und 2/1/5 inbegriffen ist.

6. Zusammensetzung gemäß Anspruch 5, **dadurch gekennzeichnet, dass** das Verhältnis Hyaluronsäure / Haferkeimlingsextrakt / L-Alanyl-L-Glutamin ein Gewichtsverhältnis von jeweils 2/1/4 ist.

7. Zusammensetzung gemäß einem der Ansprüche 1 bis 6 für ihre topische Verwendung, die zur Behandlung von Hautläsionen unter Begünstigung der Keratinozyt-Migration bestimmt ist.

8. Zusammensetzung gemäß einem der Ansprüche 1 bis 6 für ihre topische Verwendung, die zur Verbesserung der Vernarbung bestimmt ist.

9. Dermatologische oder kosmetische Zusammensetzung, umfassend als Wirkstoffprinzip eine Zusammensetzung gemäß einem der Ansprüche 1 bis 6, mit wenigstens einem dermatologisch oder kosmetisch akzeptablen Trägerstoff.

10. Zusammensetzung gemäß Anspruch 9 für ihre Verwendung bei der Behandlung von Schnittwunden, Nähten, Abschürfungen, Schrammen, Kratzwunden, Narben nach chirurgischen Eingriffen oder nach schönheitschirurgischen dermatologischen Eingriffen, oberflächlichen Verbrennungen, Sonnenbränden.

**Claims**

1. Combination comprising L-alanyl-L-glutamine, hyaluronic acid or one of the salts of same and an oat extract.

2. Combination according to claim 1, **characterised in that** the hyaluronic acid is in the form of fragments of sodium hyaluronate.

3. Combination according to claim 2, **characterised in that** the molecular weight of the fragments of hyaluronate is comprised between 50,000 and 750,000 Da.

4. Combination according to one of claims 1 to 3, **characterised in that** the oat extract is obtained from oat seedlings.

5. Combination according to claim 4, **characterised in that** the hyaluronic acid / oat seedling extract / L-alanyl-L-glutamine weight ratio is respectively comprised between 2/1/3 and 2/1/5.

6. Combination according to claim 5, **characterised in that** the hyaluronic acid / oat seedling extract / L-alanyl-L-glutamine ratio is a weight ratio respectively of 2/1/4.

7. Combination according to one of claims 1 to 6, for its topical use intended to treat skin lesions while favouring keratinocyte migration.

8. Combination according to one of claims 1 to 6, for its topical use intended to improve healing.

9. Dermatological or cosmetic composition comprising as active ingredient a combination according to one of claims 1 to 6, with at least one dermatologically or cosmetically acceptable excipient.

10. Composition according to claim 9, for its use in the treatment of cuts, sutures, grazes, scratches, scrapes, post-surgery scars or post-aesthetic dermatological interventions, superficial burns, sunburn.

**Figure unique**

## RÉFÉRENCES CITÉES DANS LA DESCRIPTION

**Documents brevets cités dans la description**

- WO 2010054879 A

**Littérature non-brevet citée dans la description**

- **SANTORO MM. ; GAUDINO G.** Cellular and molecular facets of keratinocyte reepithelization during wound healing. *EXP. CELL. RES.,* 2005, vol. 304 (1), 274-286
- **WERNER S. ; GROSE R.** Regulation of wound healing by growth factors and cytokines. *PHYSIOL. REV.,* 2003, vol. 83 (3), 835-870
- **STEFFENSEN B. ; AKKINEN L. ; LARIAVA H.** Proteolytic events of wound healing-coordinated interactions among matrix metallopteinases (MMPs), integrins, and extracellular matrix molecules. *CRIT. REV. ORAL BIOL. MED.,* 2001, vol. 12 (5), 373-398

- **MCMILLAN J.R. ; AKIYAMA M. ; SHIMIZU H.** Epidermal basement membrane zone component : ultrastructural distribution and molecular interactions. *J DERM SC.,* 2003, vol. 31, 169-177
- **SZABO I. ; WETZEL M.A. ; ROGERS TJ.** Cell-Density-Regulated Chemotactic Responsiveness of Keratinocytes In Vitro. *J INVEST DERMATOL,* 2001, vol. 117, 1083-1090